Europäisches Patentamt

European Patent Office

Office européen des brevets

(19)

(11) Publication number: **0 105 814**
**A2**

(12) # EUROPEAN PATENT APPLICATION

(21) Application number: **83420145.1**

(22) Date of filing: **05.09.83**

(51) Int. Cl.³: **A 61 L 2/10**

(30) Priority: **08.09.82 JP 135369/82 U**

(43) Date of publication of application:
**18.04.84 Bulletin 84/16**

(84) Designated Contracting States:
**AT BE CH DE FR GB IT LI LU NL SE**

(71) Applicant: **Kusakari, Hiroko**
**6371-98 Igarashi Ichinocho**
**Niigata-shi Niigata-ken(JP)**

(72) Inventor: **Kusakari, Hiroko**
**6371-98 Igarashi Ichinocho**
**Niigata-shi Niigata-ken(JP)**

(74) Representative: **Maureau, Bernard**
**CABINET GERMAIN & MAUREAU 20, boulevard E.**
**Deruelle B.P. 11**
**F-69392 Lyon Cédex 06(FR)**

(54) **An apparatus for cleanly preserving toothbrushes.**

(57) An apparatus for cleanly preserving toothbrushes comprises a preservation chamber (2) and a machine chamber (4). The preservation chamber (2) includes a sterilization lamp (5), a dry hot air blowing hole (10), a door (3) for taking the toothbrushes in or out, means (12) for supporting toothbrushes in vertical position and an exhaust port (13). The machine chamber (4) includes a fan heater (20) for blowing dry hot air, a fan motor (15) and a time switch (16).

FIG. 1

Croydon Printing Company Ltd.

EP 0 105 814 A2

1

"AN APPARATUS FOR CLEANLY PRESERVING TOOTHBRUSHES"

The present invention relates to an apparatus for cleanly preserving toothbrushes.

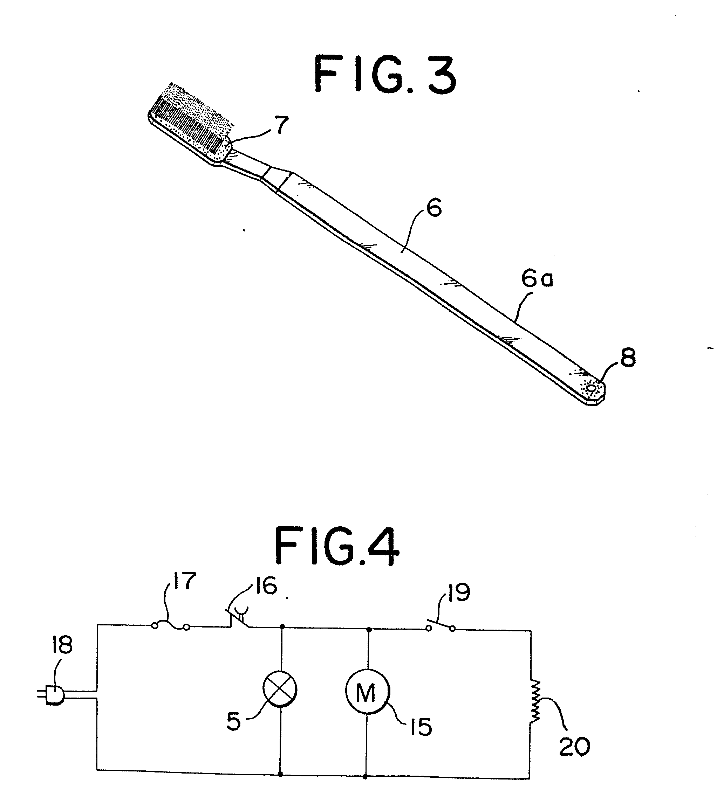

Sometimes, we find that the toothbrush used every day gets mildewed at its brush portion (7) and an end portion (8) of a stem (6a) of the toothbrush (6) as shown in Fig. 3 and becomes unpleasant. Since toothbrushes after use are generally placed near the humid toilet room or lavatory and they are used in relatively busy, early morning, cutting water and washing after use thereof become frequently imperfect, and since the toothbrushes with bacteria of oral cavity are kept in humid places, they get mildewed inevitably.

Hitherto, an apparatus for cleanly preserving toothbrushes has not been developed.

It is an object of the invention to eliminate such insanitary conditions of the toothbrushes.

According to the invention, there is provided an apparatus for cleanly preserving toothbrushes comprising a preservation chamber for toothbrushes and a machine chamber which includes means for blowing dry hot air, said preservation chamber including a sterilization lamp, a dry hot air blowing hole, a door for taking the toothbrushes in or out, means for supporting toothbrushes having holes for supporting toothbrushes in vertical position and for ventilating air and an exhaust port, whereas said machine chamber including a fan heater for blowing dry hot air, a fan motor and a time switch.

Other features and advantages of the invention will be apparent from the following description taken in connection with the accompanying drawings wherein one embodiment is illustrated by way of example.

Fig. 1 is a front perspective view showing one embodiment of the invention in which the door is opened.

Fig. 2 is a partially cut away rear view of the apparatus shown in Fig. 1.

Fig. 3 is a perspective view of a toothbrush illustrating the portions on which bacteria is likely to breed.

Fig. 4 is a circuit diagram of the apparatus.

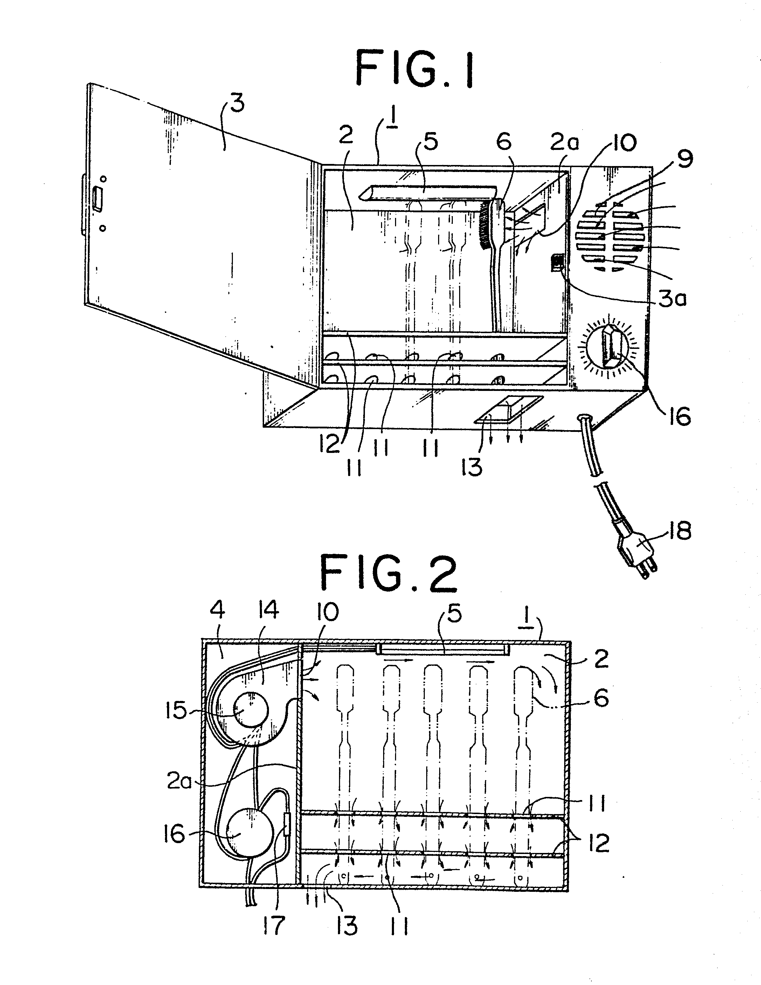

Referring now to Fig. 1 the apparatus for cleanly preserving toothbrushes according to the invention comprises a preservation chamber (2) which cleanly preserves toothbrushes in one casing, and a machine chamber (4) which includes means for blowing dry hot air or similar means.

At the ceiling of the preservation chamber (2) a sterilization lamp (5) such as ultraviolet lamp is disposed and at the upper protion of a partition (2a) between the preservation chamber (2) and the machine chamber (4) a dry hot air blowing hole (10) is arranged. The preservation chamber (2) comprises at its lower portion toothbrush supporting boards (12) in two stages, each of which has holes (11) for vertically supporting toothbrushes (6) and ventilating air. And at the bottom portion of the preservation chamber (2) an exhaust port (13) is arranged and in front of the chamber (2) there is provided a door (3) for taking the toothbrushes (6) in or out. Preferably, the door (3) can be firmly closed by a magnet (3a) secured to the partition (2a). Also preferably on the bottom of the preservation chamber a dish (not shown) for receiving droplets from the toothbrushes may be disposed.

Whereas, the machine chamber (4) includes devices for clean preservation of toothbrushes after use, that is a fan heater (20), a fan motor (15) for rotating a fan (not shown) to blow dry hot air, a time switch (16), a thermal fuse (17) for preventing over heat of the fan heater (20), and a thermostat (19) for controlling the temperature in the preservation chamber (2). Preferably, the fan heater (20) may be ceramic heater such as $BaTiO_3$ type semiconductor.

Thus the toothbrushes (6) contained in the apparatus (1) according to the invention are kept always in dry condition. And since the bacteria remaining in portions (7, 8) on the toothbrushes can be sterilized by the ultraviolet lamp (5), the toothbrushes are maintained clean in an excellent condition.

Further, referring to Figs. 1-4, an embodiment of the invention will now be explained. Toothbrushes (6) washed with water after use are inserted into the holes (11) of the supporting boards (12) in the preservation chamber (2) to maintain the toothbrushes in vertical position, said holes serving as support of the toothbrushes and as air ventilation, and then the door (3) is closed. Subsequently, when the time switch (15) is turned on by setting at desired time, the sterilization lamp (5) lights and simultaneously the fan heater (20) and the fan motor (15) are operated to introduce ambient air into the fan heater. Hot air heated by the fan heater is blown from the blow hole (10) into the preservation chamber (2) as dry hot air while the hot air removes moisture in the preservation chamber (2) and is exhausted from an exhaust port (13) through the holes (11) of the two-stage supporting boards (12) for supporting toothbrushes and ventilating air. Then the atmosphere in the preservation chamber (2) is gradually dried and sterilized by the aid of the sterilization lamp (5), and the toothbrushes (6) in the chamber (2) can be preserved always clean.

Since the toothbrushes (6) preserved clean in the apparatus (1) according to the invention are much more clean than those preserved in ordinary manner, bacteria cannot enter into the oral cavity through the toothbrushes, and diseases in the oral cavity such as pyorrhea alveolaris can be prevented, and further the apparatus of the invention can also exibit superior effect for the prevention of the infection of influenza. In addition, the apparatus for cleanly preserving toothbrushes according to the invention can be installed in the usual wash stand of lavatory.

Example :

The experimental process for determining bacterial contamination of toothbrush was as follows :

4

Brush portion of a toothbrush was cut off with a sterilized cutter knife and introduced into a sterilized ground stopper bottle containing 1 000 ml of a sterilized physiological saline solution. The resulting mixture was shaked 1 000 times to afford an original liquor which was then incubated at 37°C for a period of three days on a heart-infusion agar medium according to a multiple dilution (ten times) method and finally the number of bacteria on the agar medium was counted.

(1) The numer of bacteria on the toothbrush used every day.

65 toothbrushes were collected and the number of bacteria whereon was counted under the above experimental conditions, whereby the bacterial population of bacteria which had been adherent to the brush portion of each toothbrush was calculated. The result is summarized on the following Table 1.

Table 1 : Bacterial population of bacteria on 65 toothbrushes

| Number of bacteria | Number of toothbrushes (%) |
|---|---|
| Less than 1,000 | 16 (24.6) |
| 1,000 - <10,000 | 7 (10.8) |
| 10,000 - <100,000 | 6 (9.2) |
| 100,000 - <1,000,000 | 10 (15.4) |
| 1,000,000 - <10,000,000 | 13 (20.0) |
| 10,000,000 - <100,000,000 | 6 (9.2) |
| 100,000,000 or more | 7 (10.8) |
| Total | 65 (100) |

It was clear from the result as summarized above that about 25 % of the entire toothbrushes were contaminated with less than 1000 bacteria and 40 % of the total toothbrushes were contaminated with more than one million bacteria. Toothbrushes just after use for about one week were generally clean and several toothbrushes which had been washed with hot water after use were contaminated with only less than about 1000 bacteria. It was found that the bacterial contamination of toothbrushes was unexpectedly remarkable.

(2) With the use of an apparatus for cleanly preserving toothbrushes according to this invention, the following test result for the sterilization and drying of toothbrushes was given.

15 new toothbrushes were immersed for one hour in "rinsed water (gargled water)" containing 430,000,000/ml of buccal bacteria and then removed therefrom to count the number of bacteria on the first five toothbrushes (as control) as they were. Simultaneously, the second five toothbrushes were merely hot air (50°C) dried for a period of 20 minutes in the sterilization lamp free apparatus preceding to counting the number of bacteria thereon. The third five toothbrushes were sterilized and hot air (50°C) dried for a period of 20 minutes in the apparatus provided with the sterilization lamp prior to the counting of the number of bacteria thereon. The result of the average number of bacteria for every 5 toothbrushes was summarized below.

Table 2 : The advantageous effect due to the present apparatus for cleanly preserving toothbrushes

|  | Average number of bacteria for 5 toothbrushes |
|---|---|
| Control | 898,000 |
| No sterilization lamp | 16,000 |
| Sterilization lamp | 100 |

In case where the apparatus fitted with the sterilization lamp was used, five toothbrushes were contaminated on the average with 100 bacteria, but four toothbrushes did not exhibit bacteria and the remaining one toothbrush was contaminated with 500 bacteria. In conclusion, it was proved that the toothbrushes could suprisingly be preserved clean by the apparatus for the clean preservation of this invention.

6

## - CLAIMS -

1.- An apparatus for cleanly preserving toothbrushes comprising a preservation chamber (2) for toothbrushes and a machine chamber (4) which includes means for blowing dry hot air, said preservation chamber (2) including a sterilization lamp (5), a dry hot air blowing hole (10), a door (3) for taking the toothbrushes in or out, means (12) for supporting toothbrushes having holes (11) for supporting toothbrushes in vertical position and for ventilating air and an exhaust port (13), whereas said machine chamber (4) including a fan heater (20) for blowing dry hot air, a fan motor (15) and a time switch (16).

2.- Apparatus according to claim 1, wherein said sterilization lamp (5) is ultraviolet lamp.

3.- Apparatus according to claim 2, wherein a thermostat (19) is connected in series to the fan heater (20) to control the remperature in the preservation chamber (2).

4.- Apparatus according to claim 2, wherein a thermal fuse (17) is connected in series to a power source to prevent over heat of the fan heater (20).

5.- Apparatus according to any one of claims 2-4, wherein on the bottom of the preservation chamber (2) there is disposed a dish which receives droplets from the toothbrushes.

6.- Apparatus according to any one of preceding claims, wherein said fan heater (20) is of $BaTiO_3$ type semiconductor.

# FIG.I

# FIG.2

# FIG.3

7

6

6a

8

# FIG.4

17    16    19

18

5    M    15

20